Europäisches Patentamt

**European Patent Office**

**Office européen des brevets**

(11) Publication number: **0 169 729**
**A2**

(12) # EUROPEAN PATENT APPLICATION

(21) Application number: **85305238.9**

(22) Date of filing: **23.07.85**

(51) Int. Cl.⁴: **G 01 N 33/577**
**G 01 N 33/566**

(30) Priority: **23.07.84 US 633543**

(43) Date of publication of application:
**29.01.86 Bulletin 86/5**

(84) Designated Contracting States:
**BE DE FR GB IT NL SE**

(71) Applicant: **Becton, Dickinson and Company**
**Mack Centre Drive**
**Paramus New Jersey 07652(US)**

(72) Inventor: **Brodsky, Frances M.**
**734 Alvarado Court**
**Stanford California(US)**

(74) Representative: **Ruffles, Graham Keith et al,**
**MARKS & CLERK 57-60 Lincoln's Inn Fields**
**London WC2A 3LS(GB)**

(54) **Recovery of cell receptors.**

(57) A method is provided for recovering cell surface receptors for a specific ligand. In the method cells are incubated in the presence of a ligand for a period of time sufficient to permit the ligand to bind to the receptor on the cell membrane surface and to form a complex of the ligand and the receptor. The incubation is continued for a time sufficient to permit the complex to become internalized in the cell in the form of coated vesicles. The membrane of the cells is then disrupted prior to the time that the complex is released from the coated vesicle within the cell to provide a dispersion of cell fragments and coated vesicles. The dispersion is then contacted with a monoclonal antibody which is specific for the protein coating of the coated vesicle so as to recover the coated vesicle containing the cell surface receptor for the specific ligand.

EP 0 169 729 A2

Croydon Printing Company Ltd

Folio: 799P50646    RECOVERY OF CELL RECEPTORS

## Field of the Invention

Generally, the present invention is directed to a method for recovering cell surface receptors for a specific ligand. More particularly, the present invention is directed to the recovery of a specific cell surface receptor for use as an immunogen for producing monoclonal antibodies and for testing the efficacy of drug delivery via cell surface receptors.

## Background of the Invention

It is known that various ligands, including hormones, such as insulin; nutritional substances, such as low density lipoproteins (LDL); viruses, such as adenovirus; toxins, such as diptheria toxin and ricin; and immunoglobulin, including polyclonal antibodies and monoclonal antibodies, bind to specific receptors on the surfaces of cells. Such cell surface receptors are often markers for specific types of cells or functional lymphocyte subsets. For example, the asialoglycoprotein receptor is found only on parenchymal cells of the liver and transferrin receptor is a marker for activated lymphocytes; see, for example, Wall D. A. et al (1981) Galactose-specific Recognition System of Mammalian Liver: Receptor Distribution on the Hepatocyte Cell Surface; J. Cell Biol: 90:687-696 and Trowbridge, I.S. et al (1981) Human cell surface glycoprotein related to cell proliferation is the receptor for transferrin; Proc. Nat'l Acad. of Science U.S.A. 78:3039-3043.

Monoclonal antibodies reacting with such receptors provide both tissue specific probes and a tool for analyzing the level of lymphocyte subpopulations.

-2-

Tissue specific monoclonal antibodies can be used to identify the tissue origin of a metastatic cell and to target drugs to a specific tissue. Monoclonal antibodies which can detect lymphocyte subsets are useful in diagnosis of immunological states and also for targeting drugs to malignant cell subpopulations. Heretofore, monoclonal antibodies have been prepared which are specific to certain cell surface receptors. The current technology for preparing such monoclonal antibodies involves immunizing mice with whole cells. Of course, the whole cell contains receptors other than the receptors of interest. This complicates the screening process to recover a monoclonal antibody which is specific for the cell surface receptor of interest. The cell surface receptor of interest is a minor component of the total cell surface protein immunogen. It would be desirable to provide a purified or partially purified receptor for use in immunizing mice to simplify the procedure for producing monoclonal antibodies specific for the desired cell surface receptor.

It has recently been discovered that some cell surface receptors actually enter the cells in combination with the ligand which recognizes the receptor; Scientific American, How Receptors Bring Proteins and Particles Into Cells, A. Dautry-Varsat et. al., Vol. 250, No. 5, pp. 52-58, May, 1984. For example, it has been learned that insulin receptors and insulin enter cells together. The method whereby receptors are internalized within the cells has also recently been discovered. Some receptors are

- 3 -

localized to places on the cell surface called coated pits where they bind to a ligand, such as LDL. Other receptors bind ligands, such as insulin, then move to coated pits. The receptor and its ligand are then internalized by the cell in a membrane sack called a coated vesicle. The coated vesicles fuse and accumulate in sacks inside the cells, known as "endosomes" or "receptosomes". The coated vesicles have a coating of protein which dissociates as they form the endosomes. The endosome evolves into a form referred to as a CURL (compart for uncoupling receptor and ligand) which contains an acid bath where the ligand is released from the receptor. The ligand, in many cases, goes to the lysosome where it is digested. The receptor, in many cases, goes back to the cell surface.

As indicated, when the appropriate ligand (hormone, antibody or nutrient) binds to its cell surface receptor, those receptors aggregate in the membrane and are internalized along with the ligand. This process is called "receptor-mediated endocytosis" (RME). Internalization results in the formation of a coated vesicle inside the cell which contains receptor-ligand complexes at a much higher concentration than their occurence on the cell surface. The coated vesicles are surrounded by a protein which is probably involved in the mechanism of receptor-ligand internalization. As a result of work by Barbara M F Pearse of the Medical Research Council Laboratory of Molecular Biology in England, this protein when obtained from the pig is known as clathrin (Pearse, B M F. (1975) Coated vesicles from pig brain:purification and biochemical characterization; J. Mol. Biol. 97 93-98.

In accordance with the present invention, a method is provided for purifying cell surface receptors for use in cell analysis and antibody production.

Summary of the Invention

The present invention provides a method for recovering cell surface receptors for a specific ligand. In the method cells are incubated in the presence of a ligand for a period of time sufficient to permit the ligand to bind to the receptor on the cell membrane surface and to form a complex of the ligand and the receptor. The incubation is continued for a time sufficient to permit the complex to become internalized in the cell in the form of coated vesicles. The membrane of the cells is then disrupted prior to the time that the complex is released from the coated vesicle within the cell to provide a dispersion of cell fragments and coated vesicles. The dispersion is then contacted with a monoclonal antibody which is specific for the protein coating of the coated vesicle so as to recover the coated vesicle containing the cell surface receptor for the specific ligand.

The Drawings

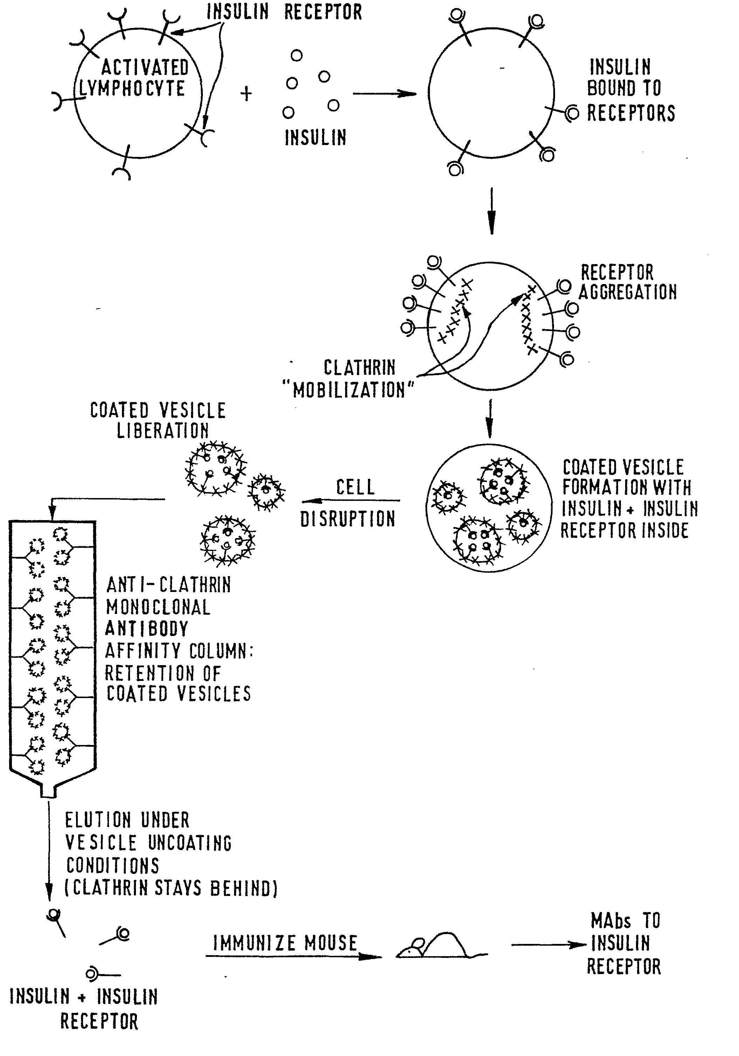

Figure 1 is a schematic diagram showing various steps in the method of the present invention for recovering cell surface receptors.

Detailed Description of the Invention

In the method of the invention, a viable culture of the cells of interest is provided. As used herein the term "cell culture" includes a viable culture of cells and a fresh tissue sample containing viable cells. The cell culture may be provided by any suitable methods, such as those described in Fresh-ney, R.I. (1983) Culture of Animal Cells: A Manual of Basic Technique Alan R. Liss, New York.

The ligand of interest is then added to the cell culture. The ligand is preferably dispersed in a suitable medium, such as phosphate buffered saline, pH 7.4. The addition of ligand, at a concentration of from about 0.1 to about 1 mg/ml, is preferably such that from about 0.001 to about 0.01 mg of ligand are added per milligram of cell.

The cells and ligand are then incubated for a period of from about 40 to about 60 minutes from about 2°C to about 6°C, preferably at about 4°C followed by incubation for about 3 to 5 minutes from about 32°C to about 42°C, preferably at 37°C. Incubation at these times and temperatures is sufficient to permit the ligand to react and bind with receptor sites to form a complex on the surface of the cell membrane and to permit the complex to become internalized within the cell in the form of a coated vesicle. The incubation conditions are selected, however, to prevent the coated vesicle from being ruptured within the cell, thereby releasing the ligand and receptor. The incubation at the lower temperature is sufficient to effect binding of the ligand to the receptor to form a complex but inhibits internalization of the complex into the cell. The incubation at the higher temperature for the indicated times is sufficient to permit internalization of the complex to form coated vesicles but is insufficient to permit the coated vesicles to evolve into endosomes.

The cell membrane is then disrupted by any suitable method to provide a dispersion of cell fragments and coated vesicles. One suitable method for disrupting the cell membrane and releasing the coated vesicle is as follows:

The cells in a solution of 10mM HEPES, pH 7.2, 0.15M NaC1, 1mM EGTA, 0.5mM $MgC1_2$, 0.02% $NaN_3$, 0.2mM PMSF are forced through a Stansted Cell Disruptor at an air pressure of about 2 bar and exposed to a back pressure of 12.5 psi. Unbroken cells and nuclei are then removed by centrifugation.

In the case of using tissue as a source of cells. The tissue would be perfused with ligand at the same concentrations, time and temperature conditions stated above. Perfusion techniques are described in Wall D.A. et al. (1981) Galactose-specific Recognition System of Mammalian Liver: Receptor Distribution on the Hepatocyte Cell Surface; J. Cell Biol: 90:687-696. The incubated tissue would be disrupted in a high speed, shear type blender, such as a Waring blender.

The coated vesicles are then recovered from the dispersion of cell fragments and coated vesicles by contacting the dispersion with a monoclonal antibody which is specific for the protein which coats the coated vesicle. There is no difficulty in developing monoclonal antibodies for such proteins, by adoption of available techiques for the preparation of monoclonal antibodies. Furthermore, in the case where this protein is clathrin, a suitable clathrin specific monoclonal antibody has been developed in mice by the Stanford University School of Medicine and is available from the American Type Culture Collection; ATCCTIB 137 CVC.4 (mouse-mouse hybridoma).

For ease of separation, the monoclonal antibody is preferably coupled to a substrate, such as a column of agarose, such as Sepharose 2B. The dispersion of cell fragments and endosomes is then flowed through the column where the protein of the coated vesicles reacts and binds to the monoclonal antibody, while the cell fragments pass through the column. Suitable means for attachment or coupling of antibodies to substrates are well known and will not be further described herein. One suitable method is described in March, S.C., Parikh, I., Cuatrecasas, P: A simplified method for cyanogen bromide activation of agarose for affinity chromatography. Anal. Biochem 60:149-152 (1974).

The coated vesicles are then removed from the column with a suitable reagent. The removal conditions are preferably selected so as to simultaneously disrupt the coated vesicle to provide a dispersion of ligand and receptor and leave the clathrin bound to the column. A suitable reagent is an aqueous solution with a pH of 8.0 containing about 1% of an octyl glucoside detergent, such as 50mM Tris-HC1. All percentages used herein are by weight unless specifically noted otherwise.

The dispersion of ligand and receptor is highly concentrated in receptor as compared to the original cell and is useful as an immunogen for production of monoclonal antibodies specific for the receptor. The receptor may also be further purified from this dispersion for use as immunogen. This involves empirical determination of further purification methods, which would vary for each individual receptor. United States Patent No. 4,364,936 to Kung et al. Column 5, line 43 to Column 6, line 39, describes a method for immunizing mice with an immunogen and producing monoclonal antibodies after such immunization.

The method of the present invention may also be used to analyze the efficacy of internalization of certain ligands, such as drugs or toxins or antibodies coupled to drugs or toxins into cells. Antibody-drug or antibody-toxin conjugates can be prepared as described by Thorpe _et al_ (1982), The preparation and cytotoxic properties of antibody-toxin conjugates, Imunol. Rev. 62:199-118. Using the method of the invention, the drug, toxin or antibody conjugate is incubated with the cells. The cells are disrupted and the coated vesicles are recovered. The coated vesicles can then be analyzed for the presence of the drug, toxin, or antibody.

The following examples further illustrate various features of the invention, but are not intended to in any way limit the scope of the invention.

Abbreviations used in Example 1 and 2:

HEPES: N-2-Hydroxyethylpiperazine-N'-2-ethanesulfonic acid

MES: 2-[N-Morpholino]ethane-sulfonic acid

EDTA: ethylene diamine tetraacetic acid

EGTA: ethylene glycol-bis (B-aminoethylether)-N,N,N',N'-tetraacetic acid

PMSF: phenylmethylsulfonyl fluoride

## Example 1

Recovery of insulin receptor

1. $10^9$ B lymphoblastoid cell line cells are suspended in 10 mls of phosphate buffered saline, pH 7.4 with 1mg/ml insulin, 4°C.

2. Cells are left on ice, 1 hr., then removed by centrifugation.

3. Cells are resuspended in 50 mls. of Buffer A (10mM HEPES pH 7.2, 0.15 M NaCL, 1mM EGTA, 0.5mM $MgCl_2$, 0.02% $NaN_3$, 0.2mM PMSF) which has been warmed previously to 37°C.

4. After 3 minutes, during which cells are filtered through gauze into a tube in a 37°C water bath, the cell suspension is passed through a Stansted Cell Disrupter at 2 bar air pressure with a back pressure of 12.5 psi, at room temperature.

5. Broken cells are collected and centrifuged 12,500 rpm in an SS-34 rotor, 4°C, for 30 minutes.

6. The supernatant is removed and spun at 100,000 xg, 4°C, to pellet coated vesicles and other membrane fragments.

7. The pellet is resuspended in 5 mls. of 20 mM MES, pH 6.2, 2mM $CaCl_2$ (Buffer B) and applied to a 1 ml column of anticlathrin monoclonal antibody coupled to Sepharose-2B, equilibrated in the same buffer.

8. After washing the column with 50 volumes of Buffer B, the column is eluted with Buffer C (50 mM Tris, pH 8.0, 1mM EDTA). Membrane vesicles are eluted off and their clathrin coat remains on the column.

9. The eluate is diluted 1:1 with 2% octylglucoside in Buffer C, to disrupt the membrane vesicles.

10. After 30 min., 4°C, the eluate is dialyzed against phosphate buffer saline, pH 7.4. This causes the receptors dissolved from the coated vesicles to form micelles.

11. Mice are immunized with the dialyzed column eluate and monoclonal antibodies to components in the eluate, including insulin receptor, are produced.

### Example 2

Determination of whether ricin-coupled to anti-transferrin receptor monoclonal antibody enters coated vesicles. (Ricin-coupled monoclonal antibody is prepared as described in Thorpe, P.E. and Ross, W.C.J. (1982), The preparation and cytotoxic properties of antibody-toxin conjugates, Immunol. Rev. 62:119-158).

1. $10^9$ B lymphoblastoid cell line cells are suspended in 10 mls. of phosphate buffered saline, pH 7.4 with 1mg/ml ricin-coupled anti-transferrin receptor mouse monoclonal antibody, 4°C.

2. Cells are treated as in Steps 2-9 above.

3. Goat-anti-mouse Ig is immobilized on a polyvinyl chloride microtiter plate and protein binding sites are blocked with bovine serum albumin.

4. Eluate from the anti-clathrin monoclonal antibody column is applied to the coated plate and after 1 hour at 4°C, unbound material is washed away.

5. The presence of mouse monoclonal antibody bound to the plate is detected with $^{125}$I-anti-mouse Ig or enzyme coupled anti-mouse Ig. A positive signal demonstrates that the ricin coupled-antibody had entered coated vesicles in the cell and would be eventually targeted to an acidic compartment in the cell.

M&C FOLIO: 799P5046                           WANGDOC: 0561C

The accompanying drawing illustrates the procedure of Example 1.

B cells are contacted with insulin and then incubated for mobilization of the coating protein and formation of vesicles. Cell disruption yields the vesicles which are temporarily retained on a column of supported monoclonal antibody. Elution under conditions favouring disruption of the vesicles leads to a dispersion containing insulin and insulin receptors, which is then used to generate monoclonal antibodies to the insulin receptor.

0169729

12

CLAIMS

1. A method for recovering cell surface receptors for a specific ligand, the method comprising incubating cells in the presence of a ligand for a period sufficient to permit the ligand to bind to a receptor on the cell membrane surface to form a complex of the ligand and the receptor and to permit the complex to become internalized in the cell in the form of a coated vesicle, the coated vesicle comprising a plurality of the complexes coated with a protein; disrupting the membrane of the cells before the complex is released from the coated vesicle within the cells, to provide a dispersion of cell fragments and coated vesicles; and contacting the dispersion with a monoclonal antibody which is specific for the protein coating of the coated vesicle so as to recover the coated vesicle containing said cell surface receptors for the ligand.

2. A method for determining the internalization of a ligand into a cell, the method comprising incubating cells in the presence of a ligand for a period of sufficient to permit the ligand to bind to a receptor on the cell membrane surface to form a complex of the ligand and the receptor to permit the complex to become internalized in the cell in the form of a coated vesicle, the coated vesicle comprising a plurality of the complexes coated with a protein; disrupting the membrane of the cells before the complex is released from the coated vesicle within the cells to provide a dispersion of cell fragments and coated vesicles; contacting the dispersion with a monoclonal antibody which is specific for the protein coating of the coated vesicle so as to recover the coated vesicle containing the cell surface receptors for the ligand; and analyzing the coated vesicle for the presence of the ligand.

3. A method in accordance with claim 1 or 2, wherein the monoclonal antibody is coupled to a substrate.

4. A method in accordance with claim 3 wherein the substate is loaded in a column and the contact of the dispersion with the monoclonal antibody is effected by flowing the dispersion through the column.

5. A method in accordance with any preceding claim, wherein the coated vesicles are disrupted to provide a dispersion of ligand and receptor.

6. A method in accordance with claim 5 wherein the

14

disperson of ligand and receptor is used as an immunogen
to provide a monoclonal antibody to the receptor.


7. A method in accordance with any preceding claim
wherein the source of cells is a tissue sample.


8. A method in accordance with claim 7 wherein the
source of cells is a viable culture of cells.


9. A method in accordance with any preceding claim,
wherein the incubation of the cells in the presence of
the ligand takes place at a first temperature of from
2°C to 4°C for a period of from 40 to 80 minutes and
thereafter takes place at a second temperature of from
32°C to 42°C for a period of from 2 to 7 minutes.


10. A method in accordance with any preceding claim,
wherein the incubation of the cells in the presence of
the ligand takes place at a first temperature of about
4°C for a time of about 60 minutes and thereafter takes
place at a second temperature of about 37°C for a period
of from about 3 to about 5 minutes.


11. A method in accordance with any preceding claim,
wherein the ligand is added to the cells as a solution

15

or dispersion having a concentration of from about 0.1 to about 1 mg/ml of ligand at a level to provide from 0.001 to about 0.01 mg of ligand per milligram of cells.

12. A method in accordance with any preceding claim, wherein the monoclonal antibody specific for the protein coating is anti-clathrin.

INSULIN RECEPTOR

ACTIVATED LYMPHOCYTE

+

INSULIN

INSULIN BOUND TO RECEPTORS

RECEPTOR AGGREGATION

CLATHRIN "MOBILIZATION"

COATED VESICLE FORMATION WITH INSULIN + INSULIN RECEPTOR INSIDE

COATED VESICLE LIBERATION

CELL DISRUPTION

ANTI-CLATHRIN MONOCLONAL ANTIBODY AFFINITY COLUMN: RETENTION OF COATED VESICLES

ELUTION UNDER VESICLE UNCOATING CONDITIONS (CLATHRIN STAYS BEHIND)

INSULIN + INSULIN RECEPTOR

IMMUNIZE MOUSE

MAbs TO INSULIN RECEPTOR